# EUROPÄISCHE PATENTSCHRIFT

(11) **EP 2 621 329 B1**
(45) Veröffentlichungstag und Bekanntmachung des Hinweises auf die Patenterteilung: **29.03.2017**
(21) Anmeldenummer: 10767924.3
(22) Anmeldetag: 30.09.2010
(51) Int. Cl.: A61B 3/10, A61B 3/00

(54) **ANORDNUNG ZUR UNTERSTÜTZUNG EINER CHIRURGISCHEN BEHANDLUNG EINES AUGES**
ARRANGEMENT FOR SUPPORTING SURGICAL TREATMENT OF AN EYE
ENSEMBLE POUR SOUTENIR UN TRAITEMENT CHIRURGICAL D'UN OEIL

(43) Veröffentlichungstag der Anmeldung: 07.08.2013
(73) Patentinhaber: WaveLight GmbH, 91058 Erlangen (DE)
(72) Erfinder: DONITZKY, Christof, 90542 Eckental/Eschenau (DE); RIEDEL, Peter, 90489 Nürnberg (DE)
(74) Vertreter: Schicker, Silvia
(86) Internationale Anmeldenummer: PCT/EP2010/005974
(87) Internationale Veröffentlichungsnummer: WO 2012/041349

(56) Entgegenhaltungen:
- EP-A1- 2 184 005
- WO-A1-87/05205
- US-A1- 2006 116 668
- US-A1- 2009 318 911
- US-A1- 2010 094 262

## Beschreibung

Die Erfindung betrifft eine Anordnung und ein Verfahren zur Unterstützung einer chirurgischen Behandlung eines Auges.

Vor einem chirurgischen Eingriff am Auge eines Patienten, beispielsweise der Implantation einer Intraokularlinse, ist es derzeit üblich, mittels eines geeigneten Diagnosegeräts Biometriedaten des Auges, wie z.B. die Vorderkammertiefe, die Linsendicke, die Augenlänge und/oder die Brechkraft der Kornea zu erfassen. Anhand der erfassten Brechkraft der Kornea kann die Achslage eines Astigmatismus berechnet werden. Basierend auf den im Rahmen der Voruntersuchung gewonnen Daten werden Marker auf das Auge, insbesondere den Limbus gesetzt, die dem Chirurgen während des Eingriffs als Positionierungshilfe beispielsweise beim Einbringen von Einschnitten oder beim Einsetzten der Intraokularlinse in das Auge dienen.

Die EP 2 184 005 A1 betrifft eine Vorrichtung zur computergestützten Augenchirurgie, die eine Verbesserung eines intraokular-chirurgischen Verfahrens durch die direkte Verknüpfung von Diagnose- und präoperativen Planungs-Ergebnissen eines Patientenauges mit dem Auge des Patienten unter dem Mikroskop des Chirurgen ermöglicht. Hierzu umfasst eine Diagnoseeinrichtung eine Kamera, die ein Referenzbild aufnimmt. Eine Datenverarbeitungseinrichtung ist eingerichtet auf Grundlage des Referenzbilds sowie Benutzereingaben das Referenzbild mit visuellen Kontextinformationen anzureichern.

Die Kontextinformationen können beispielsweise eine Schnittmarke, eine Zylinderachse, eine Pupillenmarke und/oder ein Topographiedatensatz, oder auch eine schematische Abbildung der Limbusgeometrie in Form von an den Limbus angepassten Winkelmarken enthalten. Ein Chirurgiemikroskop nimmt Livebilder des Patientenauges auf. Eine Überlagerungseinheit überlagert die Kontextinformation vor einem transparenten Hintergrund auf dem Livebild.

Die US 2010/094262 A1 betrifft ein chirurgisches Verfahren unter der Verwendung von bildgebenden Systemen. Eine Vorrichtung umfasst ein an ein Chirurgie-Mikroskop angesetztes Bildaufnahmemodul, um Echtzeitbilder und einen präoperative Patientendatensatz aufzunehmen. Der präoperative Patientendatensatz besteht aus einem Standbild des chirurgischen Zielbereichs. In dem Standbild können Veränderungen in der Opazität vorgenommen werden. Virtuelle chirurgische Referenzvermerke können direkt auf dem präoperativen Standbild hinzugefügt werden. Diese Referenzvermerke können aufgebaut sein aus visuellen Komponenten mit unterschiedlichen Transparenzgraden. Anschließend wird das präoperative Standbild mit einer Echtzeitvisulisierung des chirurgischen Zielbereichs verknüpft. Hierzu identifiziert der Chirurg zumindest ein bestimmtes sichtbares Merkmal innerhalb des Standbilds und verwendet dieses Merkmal, um das präoperative Standbild an der Echtzeitvisulisierung auszurichten. Das Merkmal kann ein Blutgefäß, ein sklerales Merkmal, ein eindeutiges Gefäßnetzwerk in der Sklera oder ein anderes identifizierendes Merkmal sein.

Die US 2006/116668 A1 betrifft ein System und ein Verfahren zur korrektiven Augenchirurgie, bei dem ein Paar von zu unterschiedlichen Zeitpunkten aufgenommenen Augenbildern ausgerichtet wird. Ein Referenzdatensatz umfasst gespeicherte digitale Bilddaten eines Patientenauges. Hierzu wird das Patientenauge durch Aufnahme eines ersten Videobildes mit einer Kamera abgelichtet. Der Referenzdatensatz wird dann manipuliert durch Entfernen von Pixeldaten aller Pixel, die von einem Limbus umschlossen sind, und von einem Bereich jenseits des Limbus, um einen ersten reduzierten Referenzdatensatz zu erhalten. Ein Echtzeitdatensatz wird während des Eingriffs unter Verwendung einer zweiten Kamera gesammelt, welcher digitale Bilddaten des Patientenauges in einer Chirurgie-Position enthält, die sich von der Vorchirurgie-Position des Referenzdatensatzes unterscheidet. Anschließend wird ein kombiniertes Bild angezeigt, das den Referenzdatensatz und den Echtzeitdatensatz umfasst, wobei entweder der Referenzdatensatz oder der Echtzeitdatensatz verschoben und/oder gedreht wird, bis eine passgenaue Registrierung erreicht ist.

Der Erfindung liegt die Aufgabe zugrunde, eine Anordnung und ein Verfahren zur Unterstützung einer chirurgischen Behandlung eines Auges bereitzustellen, die die Positionierung von Einschnitten und/oder Implantaten im Auge des Patienten erleichtern.

Diese Aufgabe wird durch eine Anordnung zur Unterstützung einer chirurgischen Behandlung eines Auges mit den Merkmalen des Anspruchs 1 gelöst.

Eine erfindungsgemäße Anordnung zur Unterstützung einer chirurgischen Behandlung eines Auges umfasst ein Diagnosegerät, das dazu eingerichtet ist, Augenstrukturdaten zu erfassen. Die von dem Diagnosegerät erfassten Augenstrukturdaten sind charakteristische Bilddaten des Auges oder charakteristische Bilddaten einzelner Bereiche des Auges . Ferner kann das Diagnosegerät dazu eingerichtet sein, weitere biometrische Daten des Auges, wie z.B. die Vorderkammertiefe, die Linsendicke, die Augenlänge und/oder die Brechkraft der Kornea zu erfassen, anhand derer die Achslage eines Astigmatismus berechnet werden kann. In der erfindungsgemäßen Anordnung zur Unterstützung einer chirurgischen Behandlung eines Auges kann lediglich ein Diagnosegerät zur Erfassung der erforderlichen Augenstrukturdaten und Biometriedaten zum Einsatz kommen. Falls gewünscht oder erforderlich, können jedoch auch mehrere Diagnosegeräte zur Erfassung verschiedener Augenstrukturdaten und Biometriedaten vorgesehen sein.

Die Datenverarbeitungseinheit der erfindungsgemäßen Anordnung zur Unterstützung einer chirurgischen Behandlung eines Auges ist dazu eingerichtet, auf der Grundlage der von dem Diagnosegerät erfassten Augenstrukturdaten ein Strukturbild zu erzeugen, das mindestens eine Abbildung einer charakteristischen Augenstruktur sowie mindestens eine relativ zu der Abbildung der charakteristischen Augenstruktur angeordnete Positionsmarkierung enthält. Das von der Datenverarbeitungseinheit erzeugte Strukturbild kann als auf einem geeigneten Speichermedium gespeichert und/oder in einer geeigneten Datenbank hinterlegt werden.

Ferner umfasst die erfindungsgemäße Anordnung zur Unterstützung einer chirurgischen Behandlung eines Auges eine Bilddateneinblendeeinrichtung, die dazu eingerichtet ist, das von der Datenverarbeitungseinheit erzeugte Strukturbild in ein von einem Operationsmikroskop während der Durchführung einer chirurgischen Behandlung des Auges erzeugtes Bild einzublenden. Grundsätzlich ist es möglich, das von der Datenverarbeitungseinheit erzeugte Strukturbild unmittelbar, d.h. ohne Zwischenspeicherung an die Bilddateneinblendeeinrichtung zu übermitteln. Vorzugsweise erfolgt jedoch, wie oben erläutert, eine Zwischenspeicherung des von der Datenverarbeitungseinheit erzeugten Strukturbilds, so dass das Strukturbild der Bilddateneinblendeeinrichtung vorzugsweise von einem Speicher übertragen wird. Die Übertragung des Strukturbilds an die Bilddateneinblendeeinrichtung kann über eine Kabelverbindung oder kabellos, beispielsweise über ein WLAN-Verbindung erfolgen. Alternativ dazu kann ein mobiler Datenträger, wie z.B. ein USB-Stick, mit dem darauf gespeicherten Strukturbild an die Bilddateneinblendeeinrichtung angeschlossen werden.

Bei der Einblendung des von der Datenverarbeitungseinheit erzeugten Strukturbilds in das von dem Operationsmikroskop erzeugte Bild erfolgt eine Bildüberlagerung, d.h. das von dem Operationsmikroskop erzeugte Bild bleibt zumindest in den Bereichen sichtbar, die nicht von der Abbildung der charakteristischen Augenstruktur oder der Positionsmarkierung überlagert werden. Falls gewünscht, kann die Abbildung der charakteristischen Augenstruktur und/oder die Positionsmarkierung in dem von der Datenverarbeitungseinheit erzeugten Strukturbild auch teiltransparent dargestellt sein, so dass bei der Einblendung des Strukturbilds in das von dem Operationsmikroskop erzeugte Bild die von der Abbildung der charakteristischen Augenstruktur und der Positionsmarkierung in dem Strukturbild überlagerten Bereiche des von dem Operationsmikroskop erzeugten Bilds teilweise sichtbar bleiben.

Durch die Einblendung des von der Datenverarbeitungseinheit erzeugten Strukturbilds in das von dem Operationsmikroskop erzeugte Bild liefert die erfindungsgemäße Anordnung einem Chirurgen bei der Durchführung einer chirurgischen Behandlung eines Auges Informationen darüber, wo die Positionsmarkierung relativ zu einer charakteristischen Augenstruktur platziert ist. Wenn die Positionsmarkierung beispielsweise die Lage eines in das Auge einzubringenden Einschnitts markiert, kann der Chirurg somit dem in das von dem Operationsmikroskop erzeugte Bild eingeblendeten Strukturbild entnehmen, wo der Einschnitt relativ zu einer charakteristischen Augenstruktur zu platzieren ist. Dadurch kann auf das Aufbringen von Markern auf das Auge verzichtet werden. Darüber hinaus ermöglicht das Strukturbild eine sehr genaue Platzierung der Positionsmarkierung relativ zu der Abbildung der charakteristischen Augenstruktur. Im Vergleich zu einem unmittelbar auf das Auge gesetzten Marker zeichnet sich die Positionsmarkierung in dem Strukturbild somit durch eine höhere Positionsgenauigkeit aus.

Erfindungsgemäß ist das Diagnosegerät der erfindungsgemäßen Anordnung zur Unterstützung einer chirurgischen Behandlung eines Auges dazu eingerichtet, als Augenstrukturdaten Bilddaten von Irisstrukturen, eines Irisrands und/oder der Limbusgeometrie zu erfassen. Grundsätzlich kann in der erfindungsgemäßen Anordnung zur Unterstützung einer chirurgischen Behandlung eines Auges ein Diagnosegerät zum Einsatz kommen, das dazu in der Lage ist, selektive Bilddaten von Blutgefäßen, Irisstrukturen, eines Irisrands und/oder der Limbusgeometrie zu erfassen. Alternativ dazu kann jedoch auch ein Diagnosegerät verwendet werden, das lediglich dazu in der Lage ist, ein Übersichtsbild des Auges zu erfassen, dem dann entsprechende Bilddaten von Blutgefäßen, Irisstrukturen, eines Irisrands und/oder der Limbusgeometrie zu entnehmen sind.

Vorzugsweise umfasst das Diagnosegerät eine Lichtquelle, die geeignet ist, charakteristische Augenstrukturen, wie z.B. Blutgefäße, Irisstrukturen, einen Irisrand und/oder die Limbusgeometrie zu betonen und hervorzuheben. Alternativ oder zusätzlich dazu kann auch das Operationsmikroskop eine Lichtquelle umfassen, die geeignet ist, charakteristische Augenstrukturen, wie z.B. Blutgefäße, Irisstrukturen, einen Irisrand und/oder die Limbusgeometrie zu betonen und hervorzuheben. Durch die Bestrahlung mit der Lichtquelle werden die charakteristischen Augenstrukturen betont und können somit leichter und genauer erfasst und erkannt werden. Die Lichtquelle des Diagnosegeräts und/oder des Operationsmikroskops ist vorzugsweise eine Grünlichtquelle.

Die Datenverarbeitungseinheit der erfindungsgemäßen Anordnung zur Unterstützung einer chirurgischen Behandlung eines Auges ist dazu eingerichtet, auf der Grundlage der von dem Diagnosegerät erfassten Augenstrukturdaten ein Strukturbild zu erzeugen, das als Abbildung einer charakteristischen Augenstruktur Bilddaten einer ausgewählten Irisstruktur, eines Irisrands oder der Limbusgeometrie vor einem transparenten Hintergrund enthält. Die Erzeugung des Strukturbilds in der Datenverarbeitungseinheit kann mit Hilfe einer geeigneten Bildverarbeitungssoftware erfolgen, die neben der Hintergrundgestaltung auch eine geeignete Gestaltung der Abbildung der charakteristischen Augenstruktur beispielsweise hinsichtlich der Farbgebung, der Transparenz, etc. ermöglicht.

Die Datenverarbeitungseinheit kann ferner dazu eingerichtet sein, auf der Grundlage der von dem Diagnosegerät erfassten Augenstrukturdaten ein Strukturbild zu erzeugen, das als Positionsmarkierung einen in Form eines Punkts, einer Linie oder einer Fläche ausgebildeten Marker, ein Raster und/oder eine die Lage eines Astigmatismus anzeigende Achse enthält. Insgesamt können in dem von der Datenverarbeitungseinheit erzeugten Strukturbild somit sehr viel mehr Positionsmarkierungsinformationen enthalten sein, als durch das Aufbringen von Markern unmittelbar auf das Auge übermittelt werden können.

Grundsätzlich kann die Bilddateneinblendeeinrichtung das von der Datenverarbeitungseinheit erzeugte Strukturbild lediglich starr in das von dem Operationsmikroskop erzeugte Bild einblenden. Wenn er dies wünscht, kann der Chirurg dann manuell, beispielsweise durch Drehen des Patienten und/oder durch Drehen einer Liegefläche für den Patienten, die in dem Strukturbild enthaltene Abbildung einer charakteristischen Augenstruktur, wie z.B. eines Blutgefäßes oder dergleichen mit der entsprechenden "realen" Augenstruktur in dem von dem Operationsmikroskop erzeugten Bild in Deckung bringen. Die Positionsmarkierung in dem Strukturbild ist für den Chirurgen dann besonders einfach und effizient nutzbar.

Eine bevorzugte Ausführungsform der erfindungsgemäßen Anordnung zur Unterstützung einer chirurgischen Behandlung eines Auges umfasst jedoch eine Positionierungseinrichtung zur Positionierung des eingeblendeten Strukturbilds relativ zu dem von dem Operationsmikroskop erzeugten Bild. Die Positionierungseinrichtung kann in die Bilddateneinblendeeinrichtung integriert sein und eine Größenanpassungsfunktion, d.h. eine Zoomfunktion umfassen und/oder eine Verschiebung und/oder Drehung des eingeblendeten Strukturbilds relativ zu dem von dem Operationsmikroskop erzeugten Bild ermöglichen. Die Positionierungseinrichtung kann beispielsweise über an der Bilddateneinblendeeinrichtung vorgesehene Stellglieder manuell bedienbar ausgeführt sein. Durch die Ausstattung der erfindungsgemäßen Anordnung mit einer Positioniereinrichtung kann das eingeblendete Strukturbild besonders einfach und komfortabel mit dem von dem Operationsmikroskop erzeugten Bild in Deckung gebracht werden, ohne dass es hierfür erforderlich ist, den Patienten zu bewegen.

Die erfindungsgemäße Anordnung zur Unterstützung einer chirurgischen Behandlung eines Auges kann ferner eine Einrichtung zur Erkennung einer ausgewählten Augenstruktur in dem von dem Operationsmikroskop erzeugten Bild umfassen. Ferner kann die Positionierungseinrichtung dazu eingerichtet sein, das eingeblendete Strukturbild in Abhängigkeit der erkannten ausgewählten Augenstruktur automatisch relativ zu dem von dem Operationsmikroskop erzeugten Bild zu positionieren. Beispielsweise kann die Einrichtung zur Erkennung einer ausgewählten Augenstruktur einen Eyetracker umfassen, der eine Pupille in dem von dem Operationsmikroskop erzeugten Bild des Auges sowie die aktuelle Position der Pupille erkennt. Die Positionierungseinrichtung kann dann beispielsweise dazu eingerichtet sein, das eingeblendete Strukturbild automatisch so relativ zu dem von dem Operationsmikroskop erzeugten Bild zu positionieren, dass ein als Positionsmarkierung in dem Strukturbild enthaltenes Raster auf den Mittelpunkt der Pupille zentriert wird.

Ferner ist es denkbar, als Einrichtung zur Erkennung einer ausgewählten Augenstruktur in dem von dem Operationsmikroskop erzeugten Bild eine Einrichtung einzusetzen, die die Erkennung einer der Abbildung der charakteristischen Augenstruktur in dem Strukturbild entsprechenden "realen" Augenstruktur in dem von dem Operationsmikroskop erzeugten Bild ermöglicht. Die Positionierungseinrichtung kann dann dazu eingerichtet sein, das eingeblendete Strukturbild automatisch so relativ zu dem von dem Operationsmikroskop erzeugten Bild zu positionieren, dass die Abbildung der charakteristischen Augenstruktur in dem Strukturbild mit der entsprechenden "realen" Augenstruktur in dem von dem Operationsmikroskop erzeugten Bild in Deckung gebracht wird.

Die Einrichtung zur Erkennung einer ausgewählten Augenstruktur in dem von dem Operationsmikroskop erzeugten Bild ist vorzugsweise dazu in der Lage, kontinuierlich zu arbeiten, d.h. kontinuierlich eine ausgewählte Augenstruktur in dem von dem Operationsmikroskop erzeugten Bild zu erkennen. Ferner ist die Positionierungseinrichtung vorzugsweise dazu eingerichtet, für eine kontinuierliche Nachführung des Strukturbilds in die gewünschte Position relativ zu dem von dem Operationsmikroskop erzeugten Bilds zu sorgen.

Bei einem Verfahren zur Durchführung einer chirurgischen Behandlung eines Auges werden Augenstrukturdaten erfasst. Auf der Grundlage der erfassten Augenstrukturdaten wird ein Strukturbild erzeugt, das mindestens eine Abbildung einer charakteristischen Augenstruktur sowie mindestens eine relativ zu der Abbildung der charakteristischen Augenstruktur angeordnete Positionsmarkierung enthält. Das Strukturbild wird in ein von einem Operationsmikroskop während der Durchführung einer chirurgischen Behandlung des Auges erzeugtes Bild eingeblendet.

Als Augenstrukturdaten können Bilddaten von Blutgefäßen, Irisstrukturen, eines Irisrands und/oder der Limbusgeometrie erfasst werden.

Während der Erfassung der Augenstrukturdaten kann das Auge von einer Lichtquelle bestrahlt werden, die geeignet ist, charakteristische Augenstrukturen, wie z.B. Blutgefäße, Irisstrukturen, einen Irisrand und/oder die Limbusgeometrie zu betonen und hervorzuheben. Alternativ oder zusätzlich dazu kann das Auge auch während der Durchführung der chirurgischen Behandlung des Auges von einer Lichtquelle bestrahlt werden, die geeignet ist, charakteristische Augenstrukturen, wie z.B. Blutgefäße, Irisstrukturen, einen Irirsand und/oder die Limbusgeometrie zu betonen und hervorzuheben. Vorzugsweise wird das Auge während der Erfassung der Augenstrukturdaten und/oder während der Durchführung der chirurgischen Behandlung des Auges von einer Grünlichtquelle mit Grünlicht bestrahlt.

Vorzugsweise wird auf der Grundlage der erfassten Augenstrukturdaten ein Strukturbild erzeugt, das als Abbildung der charakteristischen Augenstruktur eine Abbildung eines ausgewählten Blutgefäßes, einer ausgewählten Irisstruktur, eines Irisrands und/oder der Limbusgeometrie vor einem transparenten Hintergrund enthält.

Ferner kann auf der Grundlage der erfassten Augenstrukturdaten ein Strukturbild erzeugt werden, das als Positionsmarkierung einen in Form eines Punkts, einer Linie oder einer Fläche ausgebildeten Marker, ein Raster und/oder eine die Lage eines Astigmatismus anzeigende Achse enthält.

Bei dem Verfahren zur Durchführung einer chirurgischen Behandlung eines Auges kann der Patient und/oder eine Liegefläche für den Patienten bewegt werden, um das von dem Operationsmikroskop erzeugte Bild relativ zu dem eingeblendeten Strukturbild zu positionieren, beispielsweise um die Abbildung der charakteristischen Augenstruktur in dem Strukturbild mit der "realen" Augenstruktur in dem von dem Operationsmikroskop erzeugten Bild in Deckung zu bringen. Alternativ dazu kann jedoch auch das eingeblendete Strukturbild relativ zu dem von dem Operationsmikroskop erzeugten Bild positioniert werden. Dadurch wird eine Lageveränderung des Patienten während des chirurgischen Einriffs unnötig.

Ferner kann eine ausgewählte Augenstruktur in dem von dem Operationsmikroskop erzeugten Bild erkannt und das eingeblendete Strukturbild in Abhängigkeit der erkannten ausgewählten Augenstruktur automatisch relativ zu dem von dem Operationsmikroskop erzeugten Bild positioniert werden. Beispielsweise kann mittels eines Eyetrackers die Position einer Pupille in dem von dem Operationsmikroskop erzeugten Bild erkannt und das eingeblendete Strukturbild derart relativ zu dem von dem Operationsmikroskop erzeugten Bild positioniert werden, dass ein als Positionsmarkierung in dem Strukturbild enthaltenes Raster auf den Mittelpunkt der Pupille zentriert wird.

Ferner ist es denkbar, die Erkennung einer der Abbildung der charakteristischen Augenstruktur in dem Strukturbild entsprechenden "realen" Augenstruktur in dem von dem Operationsmikroskop erzeugten Bild vorzusehen und das Strukturbild automatisch derart relativ zu dem von dem Operationsmikroskop erzeugten Bild zu positionieren, dass die Abbildung der charakteristischen Augenstruktur in dem Strukturbild mit der entsprechenden "realen" Augenstruktur in dem von dem Operationsmikroskop erzeugten Bild in Deckung gebracht wird.

Falls gewünscht, kann eine kontinuierliche Erkennung einer ausgewählten Augenstruktur in dem von dem Operationsmikroskop erzeugten Bild und eine kontinuierliche Nachführung der Position des eingeblendeten Strukturbilds relativ zu dem von dem Operationsmikroskop erzeugten Bild in Abhängigkeit der erkannten ausgewählten Augenstruktur erfolgen.

Die Erfindung wird nun anhand der beigefügten, schematischen Zeichnungen näher erläutert, von denen
- Figur 1: eine Übersichtsdarstellung einer Anordnung zur Unterstützung einer chirurgischen Behandlung eines Auges zeigt und
- Figur 2: eine schematische Darstellung eines während der Durchführung einer chirurgischen Behandlung eines Auges von einem Operationsmikroskop erzeugten Bilds zeigt, in das ein von einer Datenverarbeitungseinheit erzeugtes Strukturbild eingeblendet ist.

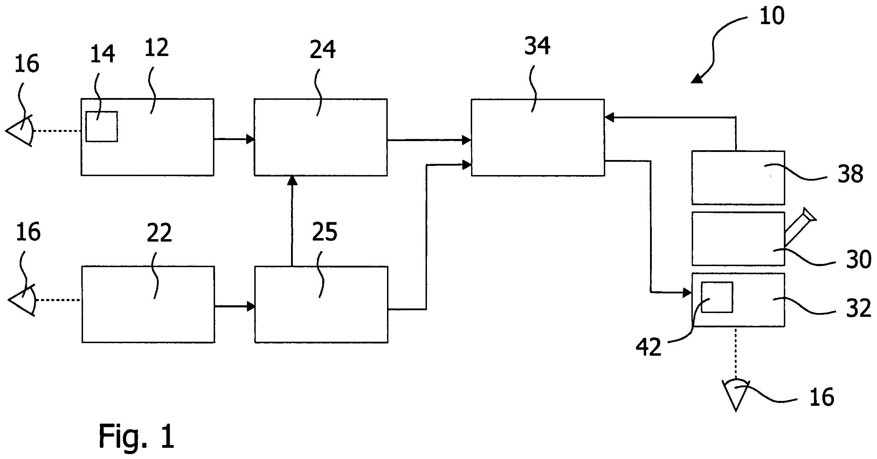

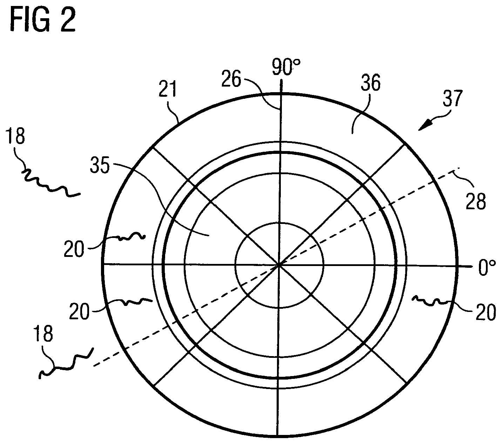

Figur 1 zeigt eine Anordnung 10 zur Unterstützung einer chirurgischen Behandlung eines Auges 16. Die Anordnung 10 umfasst ein erstes Diagnosegerät 12, das dazu eingerichtet ist, Augenstrukturdaten zu erfassen. Das erste Diagnosegerät 12 umfasst eine Grünlichtquelle 14, mit der das Auge 16 eines Patienten im Rahmen einer Voruntersuchung mit Grünlicht bestrahlt werden kann. Durch die Grünlichtbestrahlung werden im Auge 16 des Patienten vorhandene Blutgefäße 18 und Irisstrukturen 20 sowie die Limbusgeometrie 21 (siehe Figur 2) betont, so dass das erste Diagnosegerät 12 ein Bild des Auges 16 aufnehmen kann, in dem die Blutgefäße 18 und Irisstrukturen 20 sowie die Limbusgeometrie 21 gut zu erkennen sind.

Ein zweites Diagnosegerät 22 dient dazu, weitere biometrische Daten des Auges 16, wie z.B. die Vorderkammertiefe, die Linsendicke, die Augenlänge und die Brechkraft der Kornea zu erfassen, anhand derer die Achslage eines Astigmatismus berechnet werden kann. In der in Figur 1 dargestellten Anordnung 10 ist das zweite Diagnosegerät 22 separat von dem ersten Diagnosegerät 12 ausgebildet. Falls gewünscht, können die Funktionen des ersten und des zweiten Diagnosegeräts 12, 22 jedoch auch in einem einzigen Diagnosegerät integriert sein.

Das von dem ersten Diagnosegerät 12 aufgenommene Bild des Auges 16 wird an eine erste Datenverarbeitungseinheit 24 übermittelt. In dem in Figur 1 gezeigten Ausführungsbeispiel ist die erste Datenverarbeitungseinheit 24 in Form eines Personal Computers ausgebildet, auf dem eine Bildverarbeitungssoftware installiert ist. Mit Hilfe der Bildverarbeitungssoftware erzeugt die erste Datenverarbeitungseinheit 24 auf der Grundlage des von dem ersten Diagnosegerät 12 erstellten Bilds des Auges 16 ein Strukturbild, das als Abbildungen charakteristischer Augenstrukturen Abbildungen von Blutgefäßen 18, Irisstrukturen 20 und der Limbusgeometrie 21 im Auge 16 des Patienten enthält.

In ähnlicher Weise werden die von dem zweiten Diagnosegerät 22 erfassten weiteren biometrischen Daten des Auges 16 an eine zweite Datenverarbeitungseinheit 25 übermittelt. Ähnlich wie die erste Datenverarbeitungseinheit 24 ist die zweite Datenverarbeitungseinheit 25 in dem in Figur 1 gezeigten Ausführungsbeispiel in Form eines Personal Computers ausgebildet, auf dem geeignete Software zur Auswertung der erfassten Biometriedaten installiert ist. Die von der zweiten Datenverarbeitungseinheit 25 aufbereiteten Biometriedaten werden der ersten Datenverarbeitungseinheit 24 übermittelt.

Unter Berücksichtigung der der ersten Datenverarbeitungseinheit 24 von der zweiten Datenverarbeitungseinheit 25 übermittelten Biometriedaten werden nun in das von der ersten Datenverarbeitungseinheit 24 erzeugte Strukturbild Positionsmarkierungen in Form eines Rasters 26 sowie in Form einer die Lage eines Astigmatismus anzeigenden Achse 28 (siehe Figur 2) eingebracht. Das Strukturbild weist einen transparenten Hintergrund auf und wird in einer Datenbank der ersten Datenverarbeitungseinheit 24 gespeichert.

Darüber hinaus umfasst die Anordnung 10 ein Operationsmikroskop 30, das während des eigentlichen chirurgischen Eingriffs ein Bild des Auges 16 liefert. Ferner ist eine Bilddateneinblendeeinrichtung 32 vorhanden. Die Bilddateneinblendeeinrichtung 32 wird von einer ebenfalls in Form eines Personal Computers ausgebildeten Steuereinrichtung 34 gesteuert, die über eine Kabelverbindung oder eine kabellose Verbindung Zugriff auf das in der Datenbank der ersten Datenverarbeitungseinheit 24 gespeicherte Strukturbild hat. Darüber hinaus wird die Steuereinrichtung 34 von der dem zweiten Diagnosegerät 22 zugeordneten zweiten Datenverarbeitungseinheit 25 mit den von dem zweiten Diagnosegerät 22 erfassten Biometriedaten des Auges 16 beliefert. Die Datenübermittelung zwischen der zweiten Datenverarbeitungseinheit 25 und der Steuereinrichtung 34 kann ebenfalls über eine Kabelverbindung oder eine kabellose Verbindung erfolgen.

Wie in Figur 2 zu erkennen ist, ist die Bilddateneinblendeeinrichtung 32 dazu eingerichtet, während der Durchführung des eigentlichen chirurgischen Eingriffs das von der ersten Datenverarbeitungseinheit 24 erzeugte Strukturbild in das von dem Operationsmikroskop 30 erzeugte Bild des Auges 16 einzublenden. Dabei findet eine Bildüberlagerung statt, wobei die nicht von den Abbildungen der Blutgefäße 18, der Irisstrukturen 20 und der Limbusgeometrie 21 sowie dem Raster 26 und der Achse 28 überlagerten Bereiche des vom Operationsmikroskop 30 erzeugten Bilds durch die transparente Hintergrundgestaltung des Strukturbilds weiter sichtbar bleiben. Insbesondere bleiben die Pupille 35, die Iris 36 sowie die Iris umgebende Bereiche 37 des Auges 16 weiter sichtbar.

Dem von der Bilddateneinblendeeinrichtung 32 in das von dem Operationsmikroskop 30 erzeugte Bild eingeblendeten Strukturbild kann der Chirurg entnehmen, wo die Astigmatismusachse 28 relativ zu den charakteristischen Augenstrukturen, d.h. den Blutgefäßen 18, den Irisstrukturen 20 und der Limbusgeometrie 21 platziert ist. Auf der Grundlage dieser Informationen kann der Chirurg beispielsweise bestimmen, in welcher Position und Lage eine Intraokularlinse in das Auge 16 zu implantieren ist.

In dem in Figur 1 gezeigten Ausführungsbeispiel einer Anordnung 10 ist ferner eine Einrichtung 38 zur Erkennung einer ausgewählten Augenstruktur in dem von dem Operationsmikroskop 30 erzeugten Bild vorhanden. Die Einrichtung 38 umfasst einen Eyetracker, der die Position der Pupille 35 in dem Auge 16 erkennt. Von der Einrichtung 38 erfasste Daten werden der Steuereinrichtung 34 übermittelt. In Abhängigkeit der von der Einrichtung 38 erfassten Daten wird das von der Bilddateneinblendeeinrichtung 32 in das von dem Operationsmikroskop 30 erzeugte Bild eingeblendete Strukturbild mittels einer Positionierungseinrichtung 42 automatisch so relativ zu dem von dem Operationsmikroskop 30 erzeugten Bild positioniert, dass das in dem Strukturbild enthaltene Raster 26 auf den Mittelpunkt der Pupille 35 zentriert wird.

Darüber hinaus kann das Strukturbild manuell über entsprechende, in Figur 1 nicht näher veranschaulichte Stellglieder relativ zu dem von dem Operationsmikroskop 30 erzeugten Bild verschoben und/oder gedreht sowie gezoomt werden. Dadurch können die in dem Strukturbild enthaltenen Abbildungen von Blutgefäßen 18, Irisstrukturen 20 und der Limbusgeometrie 21 mit den entsprechenden "realen" Augenstrukturen in dem von dem Operationsmikroskop 30 erzeugten Bild, wie in Figur 2 gezeigt, in Deckung gebracht werden. Die in dem Strukturbild enthaltene Achse 28 kennzeichnet dann die "reale" Achslage des Astigmatismus und liefert dem Chirurgen somit wichtige Informationen bezüglich der Positionierung einer in dem Auge 16 zu implantierenden Intraokularlinse.

Bei der in Figur 1 gezeigten Anordnung 10 wird das Strukturbild manuell relativ zu dem von dem Operationsmikroskop 30 erzeugten Bild verschoben, gedreht und/oder gezoomt, um die in dem Strukturbild enthaltenen Abbildungen charakteristischer Augenstrukturen mit den entsprechenden "realen" Augenstrukturen in dem von dem Operationsmikroskop 30 erzeugten Bild in Deckung zu bringen. Alternativ dazu kann die Einrichtung 38 zur Erkennung einer ausgewählten Augenstruktur in dem von dem Operationsmikroskop 30 erzeugten Bild auch eine Einrichtung sein, die die Erkennung einer der Abbildung der charakteristischen Augenstruktur in dem Strukturbild entsprechenden "realen" Augenstruktur in dem von dem Operationsmikroskop 30 erzeugten Bild ermöglicht. Die Positioniereinrichtung 42 positioniert dann das eingeblendete Strukturbild automatisch so relativ zu dem von dem Operationsmikroskop 30 erzeugten Bild, dass die Abbildung der charakteristischen Augenstruktur in dem Strukturbild mit der entsprechenden "realen" Augenstruktur in dem von dem Operationsmikroskop 30 erzeugten Bild in Deckung gebracht wird.

## Patentansprüche

1. Anordnung (10) zur Unterstützung einer chirurgischen Behandlung eines Auges (16), mit:
- einem Operationsmikroskop (30), das dazu eingerichtet ist, während der Durchführung einer chirurgischen Behandlung des Auges (16) ein Bild mit mindestens einer Abbildung einer charakteristischen Augenstruktur zu erzeugen,
- einem Diagnosegerät (12), das dazu eingerichtet ist, Augenstrukturdaten zu erfassen, wobei das Diagnosegerät (12) dazu eingerichtet ist, als die Augenstrukturdaten Bilddaten von Irisstrukturen (20), eines Irisrands oder der Limbusgeometrie zu erfassen,
- einer Datenverarbeitungseinheit (24), die dazu eingerichtet ist, auf der Grundlage der von dem Diagnosegerät (12) erfassten Augenstrukturdaten ein Strukturbild zu erzeugen, das mindestens eine Abbildung der charakteristischen Augenstruktur sowie mindestens eine relativ zu der Abbildung der charakteristischen Augenstruktur angeordnete Positionsmarkierung enthält, und
- einer Bilddateneinblendeeinrichtung (32), die dazu eingerichtet ist, das von der Datenverarbeitungseinheit (24) erzeugte Strukturbild in das von dem Operationsmikroskop (30) während der Durchführung der chirurgischen Behandlung des Auges (16) erzeugte Bild einzublenden,
**dadurch gekennzeichnet, dass** das Strukturbild als die Abbildung der charakteristischen Augenstruktur Bilddaten einer ausgewählten Irisstruktur (20), eines Irisrands oder der Limbusgeometrie vor einem transparenten Hintergrund enthält.

2. Anordnung nach Anspruch 1, **dadurch gekennzeichnet, dass** das Diagnosegerät (12) zur Erfassung der Augenstrukturdaten und/oder das Operationsmikroskop (30) eine Grünlichtquelle (14) umfasst/umfassen.

3. Anordnung nach einem der vorhergehenden Ansprüche, **dadurch gekennzeichnet, dass** die Datenverarbeitungseinheit (24) dazu eingerichtet ist, auf der Grundlage der von dem Diagnosegerät (12) erfassten Augenstrukturdaten ein Strukturbild zu erzeugen, das als Positionsmarkierung einen in Form eines Punkts, einer Linie oder einer Fläche ausgebildeten Marker, ein Raster (26) und/oder eine die Lage eines Astigmatismus anzeigende Achse (28) enthält.

4. Anordnung nach einem der vorhergehenden Ansprüche,
**gekennzeichnet durch** eine Positionierungseinrichtung (42) zur Positionierung des eingeblendeten Strukturbilds relativ zu dem von dem Operationsmikroskop (30) erzeugten Bild.

5. Anordnung nach Anspruch 4,
**dadurch gekennzeichnet, dass** eine Einrichtung (38) zur Erkennung einer ausgewählten Augenstruktur in dem von dem Operationsmikroskop (30) erzeugten Bild vorhanden ist und dass die Positionierungseinrichtung (42) dazu eingerichtet ist, das eingeblendete Strukturbild in Abhängigkeit der erkannten ausgewählten Augenstruktur automatisch relativ zu dem von dem Operationsmikroskop (30) erzeugten Bild zu positionieren.

## Claims

1. Arrangement (10) for supporting surgical treatment of an eye (16), comprising:
- an operation microscope (30), which is designed to generate an image having at least one representation of a characteristic eye structure during the performance of a surgical treatment of the eye (16),
- a diagnostic device (12), which is designed to record eye structure data, the diagnostic device (12) being designed to record image data from iris structures (20), an iris edge or the limbus geometry as the eye structure data,
- a data processing unit (24) which is designed, on the basis of the eye structure data recorded by the diagnostic device (12), to generate a structural image which contains at least one representation of a characteristic eye structure and at least one position marking arranged relative to the representation of the characteristic eye structure, and
- an image data insertion device (32), which is designed to insert the structural image generated by the data processing unit (24) into the image generated by the operation microscope (30) during the performance of the surgical treatment of the eye (16),
**characterized in that** the structural image contains image data from a selected iris structure (20), an iris edge or the limbus geometry in front of a transparent background as the representation of the characteristic eye structure.

2. Arrangement according to Claim 1,
**characterized in that** the diagnostic device (12) for recording the eye structure data and/or the operation microscope (30) comprise(s) a green light source (14).

3. Arrangement according to one of the preceding claims, **characterized in that** the data processing unit (24) is designed, on the basis of the eye structure data recorded by the diagnostic device (12), to generate a structural image which, as position marking, contains a marker in the form of a point, a line or an area, a grid (26) and/or an axis (28) indicating the position of an astigmatism.

4. Arrangement according to one of the preceding claims,
**characterized by** a positioning device (42) for positioning the inserted structural image relative to the image generated by the operation microscope (30).

5. Arrangement according to Claim 4,
**characterized in that** there is a device (38) for detecting a selected eye structure in the image generated by the operation microscope (30), and **in that** the positioning device (42) is designed to position the inserted structural image on the basis of the detected selected eye structure automatic relative to the image generated by the operation microscope (30).

## Revendications

1. Ensemble (10) destiné à apporter une assistance dans le traitement chirurgical d'un oeil (16), comportant :
- un microscope opératoire (30), conçu pour générer une image, pendant l'exécution d'un traitement chirurgical de l'oeil (16), en formant au moins une image d'une structure oculaire caractéristique,
- un appareil de diagnostic (12), conçu pour acquérir des données de structure oculaire, dans lequel l'appareil de diagnostic (12) est conçu pour acquérir, en tant que données de structure oculaire, des données d'image de structures (20) de l'iris, d'une bordure de l'iris ou de la géométrie du limbe,
- une unité de traitement de données (24), conçue pour générer, sur la base des données de structure oculaire acquises par l'appareil de diagnostic (12), une image de structure qui contient au moins une image formée de la structure oculaire caractéristique ainsi qu'au moins un marquage de position disposé par rapport à l'image formée de la structure oculaire caractéristique, et
- un dispositif d'incorporation de données d'images (32), conçu pour incorporer l'image de structure générée par l'unité de traitement de données (24) dans l'image générée par le microscope opératoire (30) pendant l'exécution du traitement chirurgical de l'oeil (16),
**caractérisé en ce que** l'image de structure contient, en tant qu'image formée de la structure oculaire caractéristique, des données d'image d'une structure (20) sélectionnée de l'iris, d'une bordure de l'iris ou de la géométrie du limbe à l'avant d'un arrière-plan transparent.

2. Ensemble selon la revendication 1,
**caractérisé en ce que** l'appareil de diagnostic (12) destiné à acquérir les données de structure oculaire et/ou le microscope opératoire (30) comprend/comprennent une source de lumière verte (14).

3. Ensemble selon l'une des revendications précédentes, **caractérisé en ce que** l'unité de traitement de données (24) est conçue pour générer, sur la base des données de structure oculaire acquises par l'appareil de diagnostic (12), une image de structure qui contient, en tant que marquage de position, un marqueur réalisé sous la forme d'un point, d'une ligne ou d'une surface, un quadrillage (26) et/ou un axe (28) indiquant la position d'un astigmatisme.

4. Ensemble selon l'une des revendications précédentes, **caractérisé par** un dispositif de positionnement (42) destiné au positionnement de l'image de structure fusionnée par rapport à l'image générée par le microscope opératoire (30).

5. Ensemble selon la revendication 4,
**caractérisé en ce qu'**il est prévu un dispositif (38) destiné à identifier une structure oculaire sélectionnée dans l'image générée par le microscope opératoire (30) et **en ce que** le dispositif de positionnement (42) est conçu pour positionner automatique l'image de structure fusionnée par rapport à l'image générée par le microscope opératoire (30) en fonction de la structure oculaire identifiée sélectionnée.
